# EUROPEAN PATENT SPECIFICATION

(11) **EP 2 405 882 B1**
(45) Date of publication and mention of the grant of the patent: **17.06.2015**
(21) Application number: 10708871.8
(22) Date of filing: 11.03.2010
(51) Int. Cl.: A61H 35/02, A61K 9/00, A61K 47/18, A61K 47/36, A61K 47/38, A61P 27/02

(54) **COMPLEX OF POLYMERIC QUATERNARY AMMONIUM AND ANIONIC POLYMERS AS A NEW ANTIMICROBIAL AGENT FOR OPHTHALMIC COMPOSITIONS**
KOMPLEX VON POLYMEREM QUATERNÄREM AMMONIUM UND ANIONISCHEN POLYMEREN ALS NEUES ANTIMIKROBIELLES MITTEL FÜR OPHTHALMISCHE ZUSAMMENSETZUNGEN
COMPLEXE D'AMMONIUM QUATERNAIRE POLYMERE ET DE POLYMERES ANIONIQUES COMME NOUVEL AGENT ANTIMICROBIEN POUR COMPOSITIONS OPHTALMIQUES

(30) Priority: 11.03.2009 US 402184
(43) Date of publication of application: 18.01.2012
(60) Divisional application: 14182448.2
(73) Proprietor: Abbott Medical Optics Inc., Santa Ana, CA 92705 (US)
(72) Inventor: YU, Zhi-jian, Irvine CA 92620 (US)
(74) Representative: Hoffmann Eitle
(86) International application number: PCT/US2010/027017
(87) International publication number: WO 2010/105079

(56) References cited:
- WO-A1-03/053453
- WO-A1-2009/097028
- WO-A2-2006/053164
- WO-A2-2008/082948
- US-A1- 2006 251 685
- US-A1- 2009 036 404

## Description

### FIELD OF THE INVENTION

Disclosed herein are compositions and methods for eye and contact lens care. More particularly, the invention relates to ophthalmic compositions which contain a complex formed from first and second cationic components and at least one anionic polymer as antimicrobial agents for ocular care, preserving ophthalmic solutions, and/or disinfecting contact lenses.

### BACKGROUND OF THE INVENTION

The eyes are one of the most sensitive externally-exposed organs. As a result of this sensitivity or in response to degradation, the eyes may require treatment (e.g., for dry eye or infection or corrective surgery or corrective lenses). Some of these treatments include directly applying a composition to the affected eye in order to ameliorate the condition (e.g., for dry eye or infection). For treatments requiring a lens that will directly contact the eye, a composition may be needed, for example, to store, condition, rinse, or reduce the microbial load on the lens. Most, if not all, of the compositions used for eye or lens treatment require an additive with antimicrobial, disinfective, and/or preservative capabilities. The antimicrobial/preservative should be designed to cause minimal ocular irritation or user discomfort since this antimicrobial/preservative will come into contact with the eye (either directly or via a lens.) Many antimicrobials have been developed for ophthalmic use.

Antimicrobials for contact lens care are one type of ophthalmic antimicrobial. Contact lenses generally fall into three categories: hard lenses formed from materials prepared by polymerization of acrylic esters, such as polymethyl methacrylates (PMMA); rigid gas permeable (RGP) lenses formed from silicone acrylates and fluorosilicone methacrylates; and soft type lenses that may be formed from traditional copolymers such as 2-hydroxyethyl methacrylate (HEMA) or from newer silicon-containing hydrogel materials. Examples of extended wear high vapor diffusion soft contact lenses include, but are not limited to, those made from silicon-containing hydrogel materials (silicone hydrogels), such as the Focus^{®} NIGHT & DAY™ lenses from CIBA Vision (Atlanta, GA), and those made from Balafilcon^{®} A, such as PUREVISION™ lenses from Bausch & Lomb, Incorporated (Rochester, NY).

Contact lenses typically are cleaned to remove any accumulated buildup and disinfected to kill harmful microorganisms that may be present or grow on the lenses. However, ocular tissues may be impacted adversely during contact lens wear due to exposure to preservatives, disinfecting agents, cleaning agents, and other components contained in contact lens care solutions. Exposure may occur through direct tissue contact with a solution or indirect contact with a solution that may have adsorbed or absorbed to the contact lens during cleaning/disinfection and subsequently is desorbed into the eye during contact lens wear.

Soft lenses, due to their purposely designed high rate of oxygen transmission, have a tendency to bind contact lens solution ingredients. In addition, since they tend to be worn for longer periods of time, soft contact lenses are likely to desorb any bound ingredients. These desorbed materials (e.g., antimicrobial agents) may lead to, for example, ocular irritation or discomfort. Therefore, developing lens care solutions for soft contact lenses poses particular challenges.

Many multi-purpose solutions that may be used to clean, disinfect, and wet contact lenses, followed by direct insertion into the eye, are available. Multi-purpose solutions must be strong enough to kill harmful microorganisms that may be present or grow on the lenses while being gentle enough to use on the eyes. Such a solution also must be compatible with the many contact lens materials, including the silicone hydrogel materials. Measures of contact lens compatibility include contact lens discoloration, physical parameter change, fragility, and uptake/release of solution components, especially antimicrobial agents. Contact lens care solutions, such as a multi-purpose solutions (MPSs), attempt to balance cleaning and disinfection ability with safety and comfort on the eyes. The addition of more effective disinfecting agents usually has the effect of reducing contact lens material compatibility or ocular comfort of the solution. One way to achieve additional material compatibility and comfort is to reduce the amount of disinfecting agent. However, conventional knowledge dictates that this results in lower antimicrobial efficacy.

The U.S. Food and Drug Administration (FDA) requires contact lens solutions to meet certain biocidal performance criteria for destroying specified representative bacteria and fungi. Candida albicans and Fusarium saloni are the most antimicrobial agent resistant of the five representative bacteria and fungi. As a result, achieving adequate antimicrobial activity against Candida and Fusarium often is a difficult task when designing an antimicrobial component for a particular contact lens care solution. There is a need for an antimicrobial component that exhibits higher biocidal effect on Candida and Fusarium, even when used in relatively small amounts.

Aside from the FDA-specified representative bacteria and fungi, Acanthamoeba is another organism that is resistant to most antimicrobial agents. A recent increase in cyst-type Acanthamoeba infection among contact lens wearers in the U.S. indicates a need for an antimicrobial component that exhibits higher biocidal effect on Acanthamoeba.

There is need for an ophthalmic antimicrobial that exhibits broad and strong biocidal efficacy while causing minimal ocular irritation or user discomfort. The disclosed compositions and methods address this need by providing an aqueous-soluble complex formed by combining cationic antimicrobial agents or compounds and at least one anionic polymer.

WO-A-2006/053164 discloses an ophthalmic composition comprising an anionic polymer and a monomeric or dimeric antimicrobial agent.

US-A-2009/036404 discloses an ophthalmic composition comprising a carboxyl-modified fructan or a salt thereof, and a cationic antimicrobial component selected from quaternary ammonium compounds and biguanides.

WO-A-2008/082948 teaches a composition comprising alginate and a cationic antimicrobial agent such as alexidine.

### SUMMARY OF THE DISCLOSURE

An aspect of the invention is an ophthalmic composition comprising a complex formed from a first cationic component, a second cationic component and an anionic component; wherein the first cationic component comprises poly[(dimethyliminio)-2-butene-1,4-diyl chloride], α-[4-[tris(2-hydroxyethyl)ammonio]-2-butenyl]-ω-[tris(2-hydroxyethyl)ammonio]-dichloride; the second cationic component comprises a compound selected from alexidine, a salt of alexidine and cetylpyridinium chloride; the anionic component comprises hyaluronic acid; and the complex is soluble in an aqueous medium.

The complex can be dissolved in aqueous ophthalmic solutions or in an aqueous medium or both due to both lower charge load (net charge per polymer repeating unit in the complex) and lower total polymer concentration. The resulting solutions display a different antimicrobial profile than the cationic components (i.e., antimicrobial agent(s)) alone (non-complexed). The complexes display an efficacious antimicrobial profile.

In one embodiment, the hyaluronic acid (including salts such as sodium hyaluronate) is present in an amount from 0.000001% to 5% (w/w).

In one embodiment, the hyaluronic acid has a molecular weight between 10,000 and 5,000,000. In an alternate embodiment, the hyaluronic acid has a molecular weight between 600,000 and 2,000,000. In a still further embodiment, the hyaluronic acid has a molecular weight around 800,000.

In one embodiment, the second cationic compound is selected from the group consisting of alexidine and cetylpyridinium chloride (CPC), and is present in an amount ranging from 0.000001% to 5% (w/w).

In one embodiment, the composition comprises from 0.000001% to 5% (w/w) of poly[(dimethyliminio)-2-butene-1,4-diyl chloride], α-[4-[tris(2-hydroxyethyl) ammonio]-2-butenyl]-ω-[tris(2-hydroxyethyl)ammonio]-dichloride.

According to additional embodiments, the composition further comprises an ophthalmic adjuvant component selected from the group consisting of a buffer component, a viscosity-inducing component, a tonicity component, and a combination thereof.

In one embodiment, the buffer component is selected from boric acid, sodium borate, and mixtures thereof, and is present in an amount in the range of 0.01% to 2% (w/w).

In one embodiment, the viscosity-inducing component is present in an amount in the range of 0.01% to 5% (w/w).

In one embodiment, the tonicity component comprises 0.1% to 1.5% (w/w) sodium chloride.

In a further embodiment, the complex comprises poly[(dimethyliminio)-2-butene-1,4-diyl chloride], α-[4-[tris(2-hydroxyethyl) ammonio]-2-butenyl]-ω-[tris(2-hydroxyethyl)ammonio]-dichloride, alexidine, and hyaluronic acid, and the composition further comprises an aqueous medium in which the complex is dissolved at a concentration of 0.00001% to 3%.

Another aspect of the invention is an ophthalmic composition as described herein for use in a method of treating an eye, wherein the composition comprises an aqueous medium.

A further aspect of the invention is a method for treating a contact lens comprising contacting the lens with a composition as described herein, wherein the composition comprises an aqueous medium.

### DEFINITION OF TERMS

Adjuvant: As used herein, the term "adjuvant" refers to any agent that is suitable or necessary ophthalmically and is used in the ophthalmic composition to make the composition more "ophthalmically acceptable."

Antimicrobial: As used herein, the term "antimicrobial" refers to any agent or action that is biocidal, antimicrobial, antibacterial, or antifungal activity against any microbe. A skilled artisan will appreciate that antimicrobial as used herein also refers to a disinfectant or preservative (e.g., of an ocular solution).

Charge Load: As used herein, the term "charge load" refers to a molar ratio of the total charge of cationic components in a solution to the total charge of anionic components in a solution.

Ophthalmic: As used herein, the term "ophthalmic" or ophthalmic composition/solution refers to anything associated with the eyes, including compositions to treat ocular conditions (e.g., dry eye or infection) and contact lens compositions (e.g., re-wetters, disinfecting solutions, storage solutions, rinsing solutions, and multi-purpose solutions). Of course, multi-purpose contact lens care solutions are those that may be used to re-wet, disinfect, clean, store and rinse contact lenses. The antimicrobial component disclosed herein allows a user to remove a contact lens exposed to the component and place the lens directly in the user's eye for safe and comfortable wear; or, after the lens is exposed to the antimicrobial component (or a composition containing it), it may be rinsed with another quantity of the antimicrobial component (or a composition containing it) and placed in the user's eye for safe and comfortable wear.

Ophthalmically Acceptable: As used herein, the term "ophthalmically acceptable" refers to a contact lens care solution or component thereof that is compatible with ocular tissue, i.e., it does not cause significant or undue detrimental effects when brought into contact with ocular tissue.

Synergistically-effective: As used herein, the term "synergistically-effective" and "synergistically-antimicrobial" refer to any combined amount of antimicrobial compounds that exhibit synergistic biocidal, antimicrobial, antibacterial, or antifungal activity against at least one microbe. As used herein, "synergistic" and "synergistically" refer to the effect achieved with a combination of components when that effect is greater than the effect achieved with either component alone. As used herein, "synergistic" and "synergistically" includes additive effect.

Treating: As used herein, the term "treating" in reference to eyes refers to ameliorating or lessening the symptoms associated with the ocular condition (e.g., dry eye or infection) or curing the ocular condition. The term "treating" in reference to lenses refers to exposing the lens to the antimicrobial component or compositions that contain it (e.g., re-wetters, disinfecting solutions, storage solutions, rinsing solutions, and multi-purpose solutions) to, for example, re-wet, disinfect, clean, store, and rinse the lenses.

### DETAILED DESCRIPTION OF THE INVENTION

Disclosed herein are ophthalmic compositions and methods for their use, the compositions comprising a complex formed of a first cationic component, a second cationic component and an anionic polymer. The complex can be used as an antimicrobial agent or as an antimicrobial component. The antimicrobial complexes disclosed herein can provide enhanced biocidal activity over other antimicrobial agents, without significantly contributing to ocular irritation or user discomfort. Typically, when mixing hydrophilic anionic polymers with cationic polymeric antimicrobial agents, formation of precipitates occurs and the antimicrobial activity is lost. This precipitation is due to the charge load cancellation, loss of hydrophilicity, and eventual precipitation of the resulting complex. In one embodiment, both the cationic components and anionic polymers are hydrophilic, but a complexed species will result in canceled charge and a more hydrophobic species. However, it was surprisingly discovered that when both charge load and the total concentration of the components in the complexes described herein are lowered to a particular level, the precipitates can be dissolved into aqueous ophthalmic solutions. The present ophthalmic antimicrobial complexes provide a superior balance of antimicrobial activity and ophthalmic acceptability and may be used in any ophthalmic composition. Non-limiting examples of ophthalmic compositions include contact lens care compositions, re-wetting drops, treatments for eye infection, lubricating eye drops, and artificial tears.

The complex can have a synergistic antimicrobial activity against at least one microbe.

The first cationic component comprises the polyquaternary ammonium compound poly[(dimethyliminio)-2-butene-1,4-diyl chloride], α-[4-[tris(2-hydroxyethyl) ammonio]-2-butenyl]-ω-[tris(2-hydroxyethyl)ammonio]-dichloride (PQ-1; chemical registry number 75345-27-6, available under the trade names Polyquaternium 1® from Onyx Corporation, Jersey City, NJ; Onamer M®; and Polyquad®, from Alcon Laboratories, Inc., Ft. Worth, TX). This polymeric quaternary ammonium compound preferably is present in a liquid aqueous medium at a concentration of from 0.00001%, from 0.0001%, or from 0.00076%, to 0.002%, to 0.005%, to 0.01%, to 0.1%, to 1%, to 3%, or to 5% (w/w).

The second cationic component comprises a compound selected from alexidine, a salt of alexidine, and cetylpyridinium chloride. In one embodiment, alexidine has the structure of formula 1 shown below.

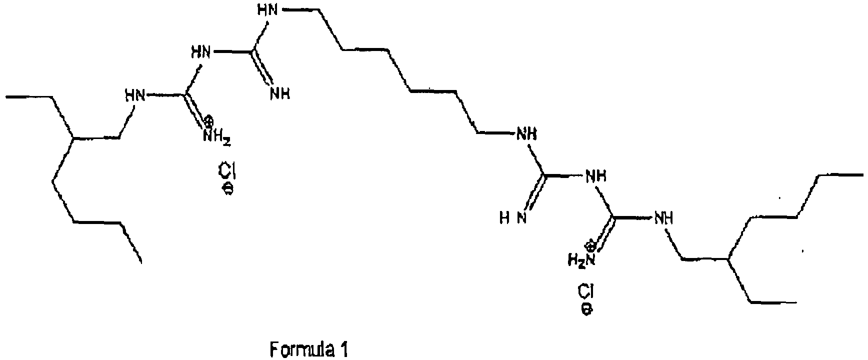

The anionic polymer can have a synergistic effect with the cationic compounds when dissolved in an aqueous ophthalmic solution. The anionic polymer is hyaluronic acid. In one embodiment, the hyaluronic acid has a molecular weight between 10,000 and 5,000,000. In an alternate embodiment, the hyaluronic acid has a molecular weight between 600,000 and 2,000,000. In a still further embodiment, the hyaluronic acid has a molecular weight around 800,000.

Contact lens materials often contain negatively charged groups, such as the carboxylate group in ACUVUE 2™ lenses by Johnson & Johnson Corporation (New Brunswick, NJ). These negative charges act to repel each other, preventing the lens from collapsing, and to attract water molecules, ensuring the lens remains well-hydrated. The adsorption of a large amount of positively charged molecules, e.g., PQ-1, will neutralize the negative charge of the lens and can cause pronounced shrinkage. The United States Food and Drug Administration criteria require contact lens care solutions not induce shrinkage in contact lens size of more than 0.2 mm. In addition, the increased molecule uptake by the lens can also increase tear osmolarity, which can cause eye discomfort. The lens will continue to adsorb positively charged molecules during repeated use of the solution. The more the positively charged molecule containing solution is used, the more positively charged molecules will accumulate on the lens and release into the eye. PQ-1 in the eye can induce cornea cell cytotoxicity, increase the osmolarity in the tears and subsequently lead to corneal damage. Complexes as described herein are very resistant to contact lens uptake due to the low relatively low charge load of the complexes as compared to the antimicrobial alone.

However, it was unexpectedly discovered that complexes of cationic compounds and at least one anionic polymer can be dissolved in aqueous ophthalmic solutions when both charge load and total concentration of the components is lowered to a certain level. This finding is contrary to common teachings in that lowering the total charge load commonly leads to the loss of the polymers' hydrophilicity. This loss of hydrophilicity leads to precipitation of antimicrobial cationic/anionic polymer complexes, and hence a loss of antimicrobial activity.

The cationic compounds used synergistically with the hyaluronic acid can be dissolved in an aqueous solution. In one embodiment, PQ-1 is used in aqueous solution at a concentration of 0.00001% to 50% (w/w). The antimicrobial components are preferably contained in a liquid aqueous medium at a total concentration of from 0.00001%, from 0.0001%, or from 0,0015%, to 0.0025%, to 0.01%, to 0.1%, to 1%, or to 3% (w/w). A skilled artisan will appreciate that hyaluronic acid and PQ-1 may be present in any synergistically-effective amount. That is, any amount of one that, when combined with a particular amount of the other, exhibits synergistic biocidal activity against at least one microbe.

Alexidine or a salt thereof can be present in aqueous solution at a concentration of 0.00001% to 50% (w/w). In one embodiment, the alexidine or salt thereof is only partially associated with the complex of PQ-1 and hyaluronic acid. A skilled artisan will appreciate that the hyaluronic acid, PQ-1 and alexidine or salt thereof may be present in any synergistically-effective amount. That is, any amount of one that, when combined with a particular amount of the others, exhibits synergistic biocidal activity against at least one microbe.

If the complex comprising the antimicrobial components is to contact the eyes (either the composition itself or via a contact lens), it is preferred that the antimicrobial components are present at ophthalmically acceptable concentrations such that ocular irritation or user discomfort are minimized or eliminated. An ophthalmic composition may comprise several ingredients and the amounts of the ingredients relative to each other may impact the ophthalmic acceptability of the composition. A skilled artisan knows how to prepare an ophthalmically acceptable composition by varying the individual amounts of ingredients.

The antimicrobial components presented in the complexes described herein may be used in compositions for treating conditions of the eye. Non-limiting examples of these compositions include eye infection treatments, lubricating eye drops, and artificial tears. Aside from an antimicrobial, these compositions typically require other components. A skilled artisan is capable of preparing numerous eye treatment compositions with the present antimicrobial components. Some eye treatment compositions may include ingredients familiar to the skilled artisan including, but not limited to a drug such as cyclosporine, polymers, salts, a lipid and oils such as polyunsaturated oils. A skilled artisan may also prepare ocular treatment complexes with the present antimicrobial components and preservatives.

Compositions herein disclosed may also include a surfactant component, preferably a nonionic surfactant, in an amount effective to clean a contact lens contacted with the composition, a buffer component in an amount effective in maintaining the pH of the composition within a physiologically acceptable range (if the composition directly will contact the eyes), an effective amount of a viscosity-inducing component, and/or an effective amount of a tonicity component. The present compositions also may include an effective amount of a chelating or sequestering component, more preferably in a range of less than 0.5% (w/v). The compositions disclosed herein preferably are ophthalmically acceptable taking into account each of the components in the concentrations employed relative to each other. In addition, each of the components preferably is employed in amounts that permit complete solubility in the compositions.

The disclosed ophthalmic compositions may be used to prepare multi-purpose compositions, that is, compositions that may be used to clean, disinfect, rinse, re-wet, and/or enhance the wearability of contact lenses. As to contact lens disinfection, it is preferred to use an amount of the antimicrobial component that reduces the microbial burden or load on the contact lens by one log order in about twenty-four hours, about six hours, more preferably in about three hours, more preferably in about one hour, more preferably in about ten minutes.

If the disclosed compositions will directly contact the eyes, it is preferred that the compositions have a pH in the physiologically acceptable range of from 4, from 5, or from 6, to 8, to 9, or to 10. In particular, the solution preferably has a pH in the range of 6 to 8. In order to achieve or maintain the desired pH, a buffer component in an amount effective to maintain the pH may be required. The buffer component may include one or more phosphate or tromethamine (TRIS, 2-amino-2-hydroxymethyl-1,3-propanediol) buffers, for example, combinations of monobasic phosphates, dibasic phosphates, and the like, or tromethamine and tromethamine hydrochloride. Particularly useful phosphate buffers are those selected from phosphate salts of alkali and/or alkaline earth metals. Examples of suitable phosphate buffers include one or more of sodium phosphate dibasic (Na₂HPO₄), sodium phosphate monobasic (NaH₂PO₄), and the corresponding potassium phosphate salts. The buffer component also may include boric acid and/or sodium borate (e.g., sodium borate 10 hydrate). The buffer component also may include an amino acid such as taurine. Buffer components typically are used in amounts from 0.01% or from 0.02%, to 0.5% (w/v) or to 2% (w/v).

The disclosed compositions may further comprise effective amounts of other components, such as a detergents or surfactants, viscosity-inducing or thickening components, chelants or sequesterants, and tonicity agents. The additional component or components may be selected from any materials known to be useful in contact lens care compositions and may be included in amounts effective to provide the desired effect or benefit. If an additional component is included, it preferably is compatible with the other components of the composition under typical use and storage conditions. For example, the additional component or components preferably do not impact adversely the antimicrobials described herein.

A surfactant may be added to the disclosed compositions to aid in cleaning, e.g., to at least aid in removing debris or deposit material from a contact lens contacted with the solution. Some exemplary surfactant(s) include, but are not limited to, nonionic surfactants (e.g., polysorbates like polysorbate 20, a.k.a. Tween® 20), 4-(1,1,3,3-tetramethylbutyl) phenol/poly(oxyethylene) polymers (e.g. Tyloxapol®), poly(oxyethylene)-poly(oxypropylene) block copolymers, and combinations of these and/or other surfactants.

Nonionic surfactants are preferred for some embodiments of compositions disclosed herein. Nonionic surfactants include poly(oxyethylene)-poly(oxypropylene) block copolymers (poloxamers), which may be obtained commercially from the BASF Corporation under the trademarks Pluronic® or Tetronic®. Pluronic® block copolymers generally can be described as polyoxyethylene/polyoxypropylene condensation polymers terminated in primary hydroxyl groups. They may be synthesized by first creating a hydrophobe of desired molecular weight by the controlled addition of propylene oxide to the two hydroxyl groups of propylene glycol or glycerin. An ethylene oxide then may be added to sandwich the hydrophobe between hydrophile groups. Tetronic® surfactants are known as poloxamines and are symmetrical block copolymers of ethylene diamine with polyoxyethylene and polyoxypropylene.

In some embodiments, the block copolymers may have average molecular weights in the range of 2500 to 30,000 Daltons, more preferably 6000 to 18,000 Daltons. Exemplary block copolymer surfactants include: poloxamer 108, poloxamer 188, poloxamer 237, poloxamer 238, poloxamer 288 poloxamer 407, Pluronic P103, Tetronic® 904, Tetronic® 1107, Tetronic® 1304 (mol. wt. 10,500), and Tetronic® 1307.

The amount of surfactant component present, if any, varies over a wide range depending on a number of factors, including, the particular surfactant(s) used, any other components in the composition, and the like. Typically, the amount of surfactant may be at least 0.005% or 0.01%, and at most 0.1%, 0.5%, or 1.0% (w/v). In another embodiment, the surfactant concentration may be from 0.05% to 0.20% (w/v).

The viscosity-inducing components employable in the present compositions preferably are those that are effective at low or reduced concentrations, are compatible with other components of the present compositions, and are nonionic. Such viscosity-inducing components may act to enhance and/or prolong the cleaning and wetting activity of any surfactant component, condition the lens surface making it more hydrophilic/less lipophilic, and/or to act as a demulcent in the eye. Increasing solution viscosity also may provide a film on the lens to facilitate comfortable wear. The viscosity-inducing component also may act to cushion the impact of contact lens insertion on the surface eye and also may serve to alleviate eye irritation.

Suitable viscosity-inducing components include, but are not limited to, water-soluble natural gums, cellulose-derived polymers and the like. Natural gums include guar gum, gum tragacanth, and the like. Cellulose-derived polymers include hydroxypropyl cellulose, hydroxypropylmethyl cellulose, methyl cellulose, hydroxyethyl cellulose, and the like. Preferred viscosity-inducing agents include cellulose derivatives (polymers), and mixtures thereof. A particularly useful viscosity-inducing component is hydroxypropylmethyl cellulose (HPMC).

The viscosity-inducing component is used in an amount effective to increase the viscosity of the solution, preferably to a viscosity in the range of 1.5 to 30 cps, or even as high as 75 cps (measured at 25°C), preferably as determined by The United States Pharmacopeial Convention (USP) Test Method No. 911 (USP 23, 1995). To achieve this range of viscosity increase, 0.01% to 5% (w/v), or 0.05% to 0.5% (w/v), of a viscosity-inducing component typically is employed.

A chelating or sequestering component preferably is included in an amount that enhances the efficacy of the antimicrobial component and/or complexes with any metal ions to more effectively clean the contact lens.

A wide range of organic acids, amines or compounds which include an acid group and an amine function are capable of acing as chelating components in the present compositions. For example, nitrilotriacetic acid, diethylenetriaminepentacetic acid, hydroxyethylethylene-diaminetriacetic acid, 1,2-diaminocyclohexane tetraacetic acid, hydroxyethylaminodiacetic acid, ethylenediamine-tetraacetic acid and its salts, polyphosphates, citric acid and its salts (e.g., trisodium citrate), tartaric acid and its salts, and the like and mixtures thereof, are useful as chelating components. Ethylenediaminetetraacetic acid (EDTA) and its alkali metal salts, are preferred, with disodium salt of EDTA, also known as disodium edetate, being particularly preferred.

The chelating component preferably is present in an effective amount, for example, in a range of 0.01% to 1% (w/v) of the solution. In a very useful embodiment, particularly when the chelating component is EDTA, salts thereof and mixtures thereof, a reduced amount is employed, for example, in the range of less than 0.05% (w/v) or even 0.02% (w/v) or less. Such reduced amounts of chelating component have been found to be effective in the present compositions while, at the same time, providing for reduced discomfort and/or ocular irritation.

The liquid aqueous medium used in conjunction with the present compositions is selected to have no substantial deleterious effect on the lens being treated, or on the wearer of the treated lens. The liquid medium is constituted to permit, and even facilitate, the lens treatment or treatments by the present compositions. The liquid aqueous medium advantageously has an osmolality in the range of at least 200-mOsmol/kg, for example, about 300 or about 350, to 400 mOsmol/kg. The liquid aqueous medium more preferably is substantially isotonic or hypotonic (for example, slightly hypotonic) and/or is ophthalmically acceptable.

The liquid aqueous medium preferably includes an effective amount of a tonicity component to provide the liquid medium with the desired tonicity. Such tonicity components may be present in the liquid aqueous medium and/or may be introduced into the liquid aqueous medium. Among the suitable tonicity adjusting components that may be employed are those conventionally used in contact lens care products, such as various inorganic salts and non-ionic polyols. Sodium chloride (NaCl) and/or potassium chloride (KCl) and the like are very useful tonicity components, as are propylene glycol, glycerin, sorbitol, mannitol and the like. The amount of tonicity component included is effective to provide the desired degree of tonicity to the solution. Such amount may be, for example, in the range of 0.2% to 1.5% (w/v). If a combination of sodium chloride and potassium chloride is employed, it is preferred that the weight ratio of sodium chloride to potassium chloride be in the range of 3 to 6 or 8.

The composition of the invention can be used for treating an eye or a contact lens. Such methods comprise contacting an eye or a contact lens with the composition at conditions effective to provide the desired treatment to the eye or the contact lens. Contacting parameters in include, among others, temperature, pressure and time. Contacting temperature may be in the range of 0°C to 100°C, more preferably in the range of 10°C to 60°C, and still more preferably in the range of 15°C to 30°C. Contacting at about ambient temperature is typical. The contacting may occur at about atmospheric pressure. The contacting preferably occurs for a time in the range of 5 minutes or 1 hour, to 2 hours, 4 hours, 6 hours, or 12 hours or more. An eye to be treated may be contacted with the composition by, for example, directly applying it to the eye as a liquid composition (e.g., eye drops). As described herein, such a composition may include other ingredients including, but not limited to, a viscosity-enhancing agent to, for example, increase the residence time of the composition in the eye or to increase user comfort.

A contact lens can be contacted with a liquid aqueous medium by immersing the lens in the medium. During at least a portion of the contacting, the liquid medium containing the contact lens can be agitated, for example, by shaking the container containing the liquid aqueous medium and contact lens, to at least facilitate removal of deposit material from the lens. After such contacting step, the contact lens may be manually rubbed to remove further deposit material from the lens. The cleaning method can also include rinsing the lens with the liquid aqueous medium prior to returning the lens to a wearer's eye. In one embodiment, the lens can be substantially free of the liquid aqueous medium prior to returning the lens to a wearer's eye. However, the method may also be as simple as contacting a lens with a solution, and placing the lens directly in an eye either with or without removing the liquid aqueous medium prior to placing the lens in the wearer's eye.

The combinations of cationic compounds and an anionic polymer described herein can form complexes in solution rather than dissolve as individual compounds. When polyquaternium-1 (PQ1) is mixed with hyaluronic acid (HA) at a low enough concentration, a complex of PQ1-HA forms. Such a complex in solution can have antimicrobial activity that differs from a solution containing the antimicrobial agent alone (not complexed with an anionic polymer), see Table 1 below. In Table 1, the PQ1-HA complex has a much different antimicrobial activity profile against both S. marcescens and A. aureus than does the PQ-1 alone.

To test the biocidal efficacy of complexes in solutions as described herein, solutions were prepared by blending together components listed below in Tables 1-4. The flowing examples are non-limiting embodiments of compositions described herein.

### Example 1 (reference example)

Table 1 below illustrates the differing antimicrobial profiles of both a polymeric quaternary ammonium compound (in this embodiment polyquaterium-1 (PQ-1) is used) and a complex of the polymeric quaternary ammonium compound and hyaluronic acid (HA, MW 800,000) (PQ1-HA). About 3 mL of the solutions listed in Table 1 were introduced into separate lens cases containing a PUREVISION™ contact lens, and additionally, each solution was placed into a test tube with various microbial agents for six hours. All the tests were at 20°C with 0.03% organic soil.

It can be seen that the antimicrobial activities are very different between PQ1 alone and the complex of PQ1/HA. PQ1 alone has much higher activity against Sm than PQ1/HA complex when no contact lens is added to the solution. When a contact lens is added to the solution, the PQ1 alone solutions showed a significant drop in anti-Sm activity. No noticeable decrease in anti Sm activity occurs to the PQ1/HA complex solution.

The same antimicrobial behaviors of PQ1/HA complex and PQ1 also hold for anti Fs activities except the degree of difference is more profound. A solution containing PQ-1 without HA can kill more than 3 logs of Fs when no lens is involved and almost totally lost the activity when a Purevision lens is added to the solution. The significant loss of antimicrobial activity leads to two negative consequences: a large quantity of PQ1 uptake by the lens and its release afterward to the eye will cause eye toxicity and, meanwhile, weaken efficacy against microorganisms. On the other hand, the PQ/HA complex solutions show no adverse change in antimicrobial activity with and without lenses and no lens uptake of antimicrobial agent. Therefore, PQ1/HA complex is likely safer to the cornea cell and more efficacious against microbes.

The PQ1-HA complex can possess a higher antimicrobial activity against some organisms. As displayed in Table 1, after a four day inoculation with PUREVISION™ contact lenses, the PQ1-HA complex was significantly more effective against F. solani than the antimicrobial agent alone.

**Table 1**

| **Ingredients** | **%w/w** | **%w/w** | **%w/w** | **%w/w** |
|---|---|---|---|---|
| Polyquaternium-1 (PQ1) | 0.00015 | 0.0003 | 0.00015 | 0.0003 |
| Boric acid | 0.60 | 0.60 | 0.60 | 0.60 |
| Sodium Borate-10H₂O | 0.18 | 0.18 | 0.18 | 0.18 |
| NaCl | 0.40 | 0.40 | 0.40 | 0.40 |
| Ethylene diamine tetraacetic acid (EDTA) | 0.05 | 0.05 | 0.05 | 0.05 |
| Tetronic 904 | 0.10 | 0.10 | 0.10 | 0.10 |
| Pluronic F87 | 0.05 | 0.05 | 0.05 | 0.05 |
| Hyaluronic acid (HA) | - | - | 0.02 | 0.02 |
| Water | QS | QS | QS | QS |
| Microorganisms | **Log drops at 6 hours in test tube without contact lenses** | | | |
| *S*. *marcescens* | 3.50 | 3.72 | 1.36 | 1.28 |
| *F. solani* | 3.40 | | 2.94 | |
| | **Inoculated after 4 days depletion with PUREVISION™ lenses In lens Case \** | | | |
| | **Log drops at 6 hours contact time** | | | |
| *S. marcescens* | 1.0 | 1.15 | 1.31 | 1.53 |
| F. solani | 0.26 | 0.34 | 2.81 | 2.75 |

### Example 2

When PQ-1 is mixed with alexidine (ALX) or a salt thereof and HA at a low enough concentration, a complex of PQ1-ALX-HA forms. Such a complex in solution can have antimicrobial activity that differs from a solution containing the antimicrobial agent alone (not complexed with an anionic polymer). Table 2 below illustrates the differing antimicrobial profiles of both the PQ-1 and the complex of PQ1-HA with the addition of alexidine, which forms the complex PQ1-ALX-HA. About 3 mL of the solutions listed in Table 2 were introduced into separate lens cases containing a PUREVISION™ contact lens or ACUVUE 2™ contact lens, and additionally, each solution was placed into a test tube with various microbial agents for six hours; the results are displayed below in Table 2.

Comparing #2 and #3 one can see that the activities of the solution with Alexidine-PQ1 alone against F. solani are almost totally lost (from 4 log reduced to 0.2 log) when lenses are added. The activity for F. solani remains very strong for Alexidine-PQ1/HA complex solution with log drops remain about 3. The activity against S. aureus is also stronger for Alexidine-PQ1/HA complex solution with lenses than that of Alexidine-PQ1 alone solution.

**Table 2**

| **Ingredients** | **#1 %w/w** | **#2 %w/w** | **#3 %w/w** |
|---|---|---|---|
| Alexidine 2HCl | 0.0001 | 0.0002 | 0.0002 |
| PQ1 | 0.00012 | 0.00015 | 0.00015 |
| Boric acid | 0.6 | 0.6 | 0.6 |
| Sodium Borate-10H₂O | 0.18 | 0.18 | 0.18 |
| NaCl | 0.4 | 0.4 | 0.4 |
| EDTA | 0.05 | 0.05 | 0.05 |
| Tetronic 904 | 0.1 | 0.1 | 0.1 |
| PF87 | 0.05 | 0.05 | 0.05 |
| HA | 0.02 | 0.02 | |

| Microorganisms | **Log drops at 6 hours without contact lens** | | |
|---|---|---|---|
| *S*. *marcescens* | >4.9 | >4.9 | >4.9 |
| *S. aureus* | >4.94 | >4.94 | >4.94 |
| *F. solani* | 3.52 | 4.0 | 4.0 |

| | **Log drops at 6 hours after 4 day depletion with ACUVUE 2™ lenses** | | |
|---|---|---|---|
| *S. marcescens* | 4.11 | 4.89 | 4.89 |
| *S. aureus* | 3.22 | 4.3 | 2.64 |
| *F. solani* | 2.2 | 2.72 | 0.22 |

| | **Log drops at 6 hours after 4 day depletion with PUREVISION™ lenses** | | |
|---|---|---|---|
| *S*. *marcescens* | 1.88 | 2.07 | 1.95 |
| *S. aureus* | 2.91 | 3.39 | 3.2 |
| *P. solani* | 2.75 | 3.32 | 0.24 |

### Example 3

Table 3 below illustrates the differing antimicrobial profiles of both the polymeric quaternary ammonium compound PQ-1 and the complex of PQ1-HA with the addition of alexidine, which forms the complex PQ1-ALX-HA, with varying concentrations of HA added to each solution. About 3 mL of the solutions listed in Table 3 were introduced into separate lens cases containing a PUREVISION™ contact lens; the results are displayed below in Table 3.

**Table 3**

| **Ingredients** | **A (w/w)** | **B (w/w)** | **C (w/w)** | **D (w/w)** | **E (w/w)** |
|---|---|---|---|---|---|
| Alexidine 2HCl | 0.0001 | 0.0001 | 0.0001 | 0.0001 | 0.0001 |
| PQ-1 | 0.00012 | 0.00012 | 0.00012 | 0.00012 | 0.00012 |
| Boric Acid | 0.60 | 0.60 | 0.60 | 0.60 | 0.60 |
| Sodium Borate - 10H₂O | 0.18 | 0.18 | 0.18 | 0.18 | 0.18 |
| NaCl | 0.40 | 0.40 | 0.40 | 0.40 | 0.40 |
| EDTA | 0.05 | 0.05 | 0.05 | 0.05 | 0.05 |
| Tetronic 904 | 0.10 | 0.10 | 0.10 | 0.10 | 0.10 |
| Pluronic F87 | 0.05 | 0.05 | 0.05 | 0.05 | 0.05 |
| HA | 0.02 | 0.01 | 0.005 | 0.003 | |
| PQ1+Alexidine to HA Weight Ratio | 0.011 | 0.022 | 0.044 | 0.073 | |

| | **Log drops at 6 hours after 4 day depletion with PUREVISION™ lenses** | | | | |
|---|---|---|---|---|---|
| *S*. *marcescens* | 1.00 | 1.08 | 1.21 | 1.71 | 1.70 |

| **Ingredients** | **A (w/w)** | **B (w/w)** | **C (w/w)** | **D (w/w)** | **E (w/w)** |
|---|---|---|---|---|---|
| *S. aureus* | 2.77 | 2.77 | 2.89 | 2.63 | 2.08 |
| *F. solani* | 2.73 | 2.81 | 2.90 | 2.73 | 0.38 |

As may be seen, particularly with regard to the activity against F. solani and S. aureus, the activity is stronger for the Alexidine-PQ1/HA complex solution than that of Alexidine-PQ1 alone solution. One surprising result from Table 3 is that the Alexidine-PQ1/HA complex is more efficacious against S. marcescens at low HA concentration with constant Alexidine-PQ1 concentration or at high weight ratio of Alexidine-PQ1 to HA. Without wishing to be bound by theory, it is believed that the complex's anti-S. marcescens behavior is caused by the fact that, at the high weight ratio of Alexidine-PQ1 to HA, the Alexidine-PQ1 antimicrobial molecules are packed more densely in the complex molecules. It is thought that S. marcescens is more vulnerable under the simultaneous attack from several adjacent Alexidine and PQ1 molecules in the HA complex. The best antimicrobial activity can be reached for the solutions above when the weight ratio of Alexidine-PQ1 to HA is about > 0.04.

### Example 4

Table 4 below illustrates the differing antimicrobial profiles of both the polymeric quaternary ammonium compound PQ-1 and the complex of CPC-PQ1-HA with varying concentrations of PQ-1 added to each solution. About 3 mL of the solutions listed in Table 4 were introduced into separate lens cases containing a PUREVISION™ contact lens; the results are displayed below in Table 4.

**Table 4**

| Ingredients | %w/w | %w/w | %w/w | %w/w | %w/w | %w/w |
|---|---|---|---|---|---|---|
| CPC, ppm | 0.00007 | 0.00007 | 0.00012 | | 0.00012 | 0.00012 |
| PQ1 | 0.00012 | 0.00015 | 0.00015 | 0.00015 | | 0.00015 |
| Boric acid | 0.60 | 0.60 | 0.60 | 0.60 | 0.60 | 0.60 |
| Sodium Borate-10H20 | 0.18 | 0.18 | 0.18 | 0.18 | 0.18 | 0.18 |
| NaCl | 0.40 | 0.40 | 0.40 | 0.40 | 0.40 | 0.40 |
| EDTA | 0.05 | 0.05 | 0.05 | 0.05 | 0.05 | 0.05 |
| Pluronic F87 | 0.05 | 0.05 | 0.05 | 0.05 | 0.05 | 0.05 |
| HA (Novozymes) | 0.02 | 0.02 | 0.02 | | | |

| | **Log drops at 6 hours without contact lens** | | | | | |
|---|---|---|---|---|---|---|
| *S. marcescens* | 4.9 | 4.9 | 4.9 | 3.4 | 3.0 | 4.21 |
| *F. solani* | 2.92 | 3.10 | 4.00 | 4.18 | 1.71 | 2.89 |

| | **Log drops at 6 hours after 4 day depletion with ACUVUE 2™ lenses** | | | | | |
|---|---|---|---|---|---|---|
| *S*. *marcescens* | 2.25 | 2.3 | 3.52 | 0.23 | 0.21 | 0.53 |
| *F*. *solani* | 1.66 | 1.6 | 1.96 | 0.17 | -0.03 | 0.15 |

Complexes as described herein are very resistant to contact lens uptake due to the low relatively low charge load of the complexes as compared to the antimicrobial alone. In addition, the molecular size of the polymeric complex can be larger than the mesh size of the contact lens material, which can prevent the complex from being absorbed into the lens. Therefore, the solutions with complex formation described in Tables 1, 2, 3 and 4 can remain highly efficacious after many days soaking with contact lenses. Additionally, in the absence of the complex, the individual antimicrobial loses its antimicrobial activity after a couple of days soaking with the contact lenses. The loss of antimicrobial activity is much more severe for certain microbes (e.g., F. solani). Industry awareness of this issue is supported by the fact that the new FDA rules recommend that any new MPS solution should pass antimicrobial activity test after being soaked in the lens case with the presence of a contact lens for 4 days.

When comparing antimicrobial activity, when the polymeric quaternary ammonium compound concentration is held constant (see Table 3 above), the smaller the anionic polymer concentration, the higher the antimicrobial effect against S. marcescens can be. This is a surprising result, because it is commonly taught in the art that the lower the anionic polymer concentration the higher the rate of loss of the polymeric quaternary ammonium due to uptake by the contact lens. This uptake by the contact lens reduces the antimicrobial activity. However, as displayed in Table 3 above, the mere presence of the anionic polymer in the solution appears to balance the charge load thereby reducing the uptake of the antimicrobial by the contact lens.

Further, the addition of small antimicrobial agents (e.g., alexidine) to the complex thereby forming PQ1-ALX-HA, (Tables 2 and 3) can have a synergistic effect on the complex of polymeric quaternary ammonium and anionic polymer. Therefore, the use of a small antimicrobial agent to compliment the complexes described herein may be efficacious.

The terms "a," "an," "the" and similar referents used in the context of describing the invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed.

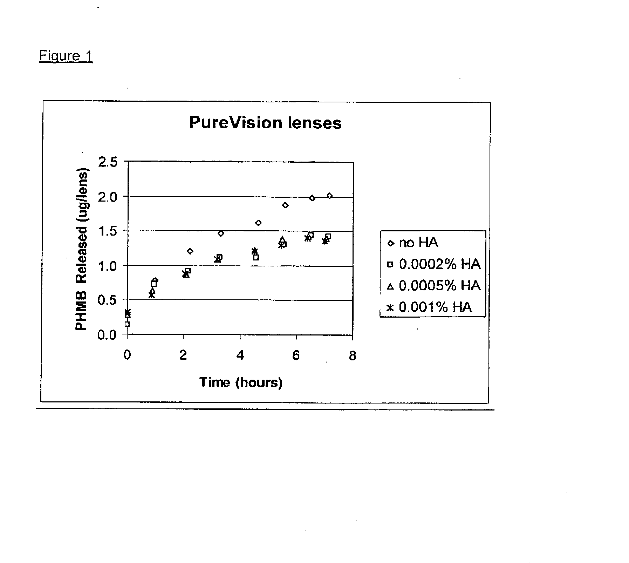

## Claims

1. An ophthalmic composition comprising a complex formed from a first cationic component, a second cationic component and an anionic component;
wherein:
the first cationic component comprises poly[(dimethyliminio)-2-butene-1,4-diyl chloride], α-[4-[tris(2-hydroxyethyl)ammonio]-2-butenyl]-ω-[tris(2-hydroxyethyl)ammonio]-dichloride;
the second cationic component comprises a compound selected from alexidine, a salt of alexidine and cetylpyridinium chloride;
the anionic component comprises hyaluronic acid; and
the complex is soluble in an aqueous medium.

2. An ophthalmic composition according to Claim 1, wherein the hyaluronic acid is present in an amount of 0.000001% to 5% (w/w).

3. An ophthalmic composition according to Claim 1 or Claim 2, wherein the second cationic component comprises alexidine or a salt thereof.

4. An ophthalmic composition according to Claim 3, wherein the alexidine or salt thereof is present in an amount of 0.000001% to 5% (w/w).

5. An ophthalmic composition according to any preceding claim, which comprises 0.000001% to 5% (w/w) of poly[(dimethyliminio)-2-butene-1,4-diyl chloride], α-[4-[tris(2-hydroxyethyl) ammonio]-2-butenyl]-ω-[tris(2-hydroxyethyl)ammonio]-dichloride.

6. An ophthalmic composition according to any preceding claim, further comprising an ophthalmic adjuvant component selected from a buffer in an amount of 0.01% to 2% (w/w), a viscosity-inducing component in an amount of 0.01% to 5% (w/w), a tonicity component comprising 0.1% to 1.5% (w/w) of sodium chloride, and a combination thereof, wherein the buffer is selected from boric acid, sodium borate and a mixture thereof.

7. An ophthalmic composition according to any preceding claim, further comprising an aqueous medium.

8. An ophthalmic composition according to any of Claims 1-6, wherein the complex comprises poly[(dimethyliminio)-2-butene-1,4-diyl chloride] ,α-[4-[tris(2-hydroxyethyl)ammoniol-2-butenyl]-ω-[tris(2-hydroxyethyl)ammonio]-dichloride, alexidine and hyaluronic acid, the composition further comprises an aqueous medium in which the complex is dissolved, and the complex is contained in the aqueous medium at a concentration of 0.00001% to 3%.

9. An ophthalmic composition as defined in Claim 7 or Claim 8 for use in a method of treating an eye.

10. A method for treating a contact lens comprising contacting the contact lens with an ophthalmic composition as defined in Claim 7 or Claim 8.

## Patentansprüche

1. Ophthalmische Zusammensetzung, umfassend einen Komplex aus einer ersten kationischen Komponente, einer zweiten kationischen Komponente und einer anionischen Komponente;
wobei:
die erste kationische Komponente Poly-[(dimethyliminio)-2-buten-1,4-diylchlorid], α-[4-[Tris(2-hydroxyethyl)ammonio]-2-butenyl]-ω-[tris(2-hydroxyethyl)ammonio]-dichlorid umfasst;
die zweite kationische Komponente eine Verbindung umfasst, die aus Alexidin, einem Salz von Alexidin und Cetylpyridiniumchlorid ausgewählt ist;
die anionische Komponente Hyaluronsäure umfasst; und
der Komplex in einem wässrigen Medium löslich ist.

2. Ophthalmische Zusammensetzung nach Anspruch 1, wobei die Hyaluronsäure in einer Menge von 0,000001% bis 5% (w/w) vorliegt.

3. Ophthalmische Zusammensetzung nach Anspruch 1 oder Anspruch 2, wobei die zweite kationische Komponente Alexidin oder ein Salz davon umfasst.

4. Ophthalmische Zusammensetzung nach Anspruch 3, wobei das Alexidin oder Salz davon in einer Menge von 0,000001% bis 5% (w/w) vorliegt.

5. Ophthalmische Zusammensetzung nach einem der vorhergehenden Ansprüche, die 0,000001% bis 5% (w/w) Poly[(dimethyliminio)-2-buten-1,4-diylchlorid], α-[4-[Tris(2-hydroxyethyl)ammonio]-2-butenyl]-ω-[tris(2-hydroxyethyl)ammonio]-dichlorid umfasst.

6. Ophthalmische Zusammensetzung nach einem der vorhergehenden Ansprüche, ferner umfassend eine ophthalmische Adjuvanskomponente, ausgewählt aus einem Puffer in einer Menge von 0,01% bis 2% (w/w), einer viskositätsinduzierenden Komponente in einer Menge von 0,01% bis 5% (w/w), einer Tonizitätskomponente, umfassend 0,1% bis 1,5% (w/w) Natriumchlorid, und eine Kombination davon, wobei der Puffer aus Borsäure, Natriumborat und einer Mischung davon ausgewählt ist.

7. Ophthalmische Zusammensetzung nach einem der vorhergehenden Ansprüche, ferner umfassend ein wässriges Medium.

8. Ophthalmische Zusammensetzung nach einem der Ansprüche 1-6, wobei der Komplex Poly[(dimethyl-iminio)-2-buten-1,4-diylchlorid], α-[4-[Tris(2-hydroxyethyl)ammonio]-2-butenyl]-ω-[tris(2-hydroxyethyl)ammonio]-dichlorid, Alexidin und Hyaluronsäure umfasst, die Zusammensetzung ferner ein wässriges Medium umfasst, in dem der Komplex gelöst ist, und der Komplex in dem wässrigen Medium in einer Konzentration von 0,00001% bis 3% enthalten ist.

9. Ophthalmische Zusammensetzung gemäß Anspruch 7 oder Anspruch 8 zur Verwendung bei dem Verfahren zur Behandlung eines Auges.

10. Verfahren zur Behandlung einer Kontaktlinse, bei dem man die Kontaktlinse mit einer ophthalmischen Zusammensetzung gemäß Anspruch 7 oder Anspruch 8 in Berührung bringt.

## Revendications

1. Composition ophtalmique comprenant un complexe formé à partir d'un premier composant cationique, d'un deuxième composant cationique et d'un composant anionique ;
dans laquelle :
le premier composant cationique comprend du poly[chlorure de (diméthyliminio)-2-butène-1,4-diyle], α-[4-[tris(2-hydroxyéthyl)ammonio)-2-butényl]-ω-[tris(2-hydroxyéthyl)ammonio]-dichlorure ;
le deuxième composant cationique comprend un composé choisi parmi l'alexidine, un sel d'alexidine et le chlorure de cétylpyridinium ;
le composant anionique comprend de l'acide hyaluronique ; et
le complexe est soluble dans un milieu aqueux.

2. Composition ophtalmique selon la revendication 1, dans laquelle l'acide hyaluronique est présent dans une quantité de 0,000001 % à 5 % (m/m).

3. Composition ophtalmique selon la revendication 1 ou la revendication 2, dans laquelle le deuxième composant cationique comprend de l'alexidine ou un sel de celle-ci.

4. Composition ophtalmique selon la revendication 3, dans laquelle l'alexidine ou le sel de celle-ci est présent dans une quantité de 0,000001 % à 5 % (m/m).

5. Composition ophtalmique selon une quelconque revendication précédente, qui comprend 0,000001 % à 5 % (m/m) de poly[chlorure de (diméthyliminio)-2-butène-1,4-diyle], α-[4-[tris(2-hydroxyéthyl)ammonio)-2-butényl]-ω-[tris(2-hydroxyéthyl)ammonio]-dichlorure.

6. Composition ophtalmique selon une quelconque revendication précédente, comprenant en outre un composant adjuvant ophtalmique choisi parmi un tampon dans une quantité de 0,01 % à 2 % (m/m), un composant induisant une viscosité dans une quantité de 0,01 % à 5 % (m/m), un composant de tonicité comprenant 0,1 % à 1,5 % (m/m) de chlorure de sodium, et une combinaison de ceux-ci, le tampon étant choisi parmi l'acide borique, le borate de sodium et un mélange de ceux-ci.

7. Composition ophtalmique selon une quelconque revendication précédente, comprenant en outre un milieu aqueux.

8. Composition ophtalmique selon l'une quelconque des revendications 1 à 6, dans laquelle le complexe comprend du poly[chlorure de (diméthyliminio)-2-butène-1,4-diyle], α-[4-[tris(2-hydroxyéthyl)ammonio)-2-butényl]-ω-[tris(2-hydroxyéthyl)ammonio]-dichlorure, de l'alexidine et de l'acide hyaluronique, la composition comprenant en outre un milieu aqueux dans lequel le complexe est dissous, et le complexe est contenu dans le milieu aqueux à une concentration de 0,00001 % à 3 %.

9. Composition ophtalmique telle que définie dans la revendication 7 ou la revendication 8 pour utilisation dans un procédé de traitement d'un oeil.

10. Procédé de traitement d'une lentille de contact comprenant la mise en contact de la lentille de contact avec une composition ophtalmique telle que définie dans la revendication 7 ou la revendication 8.
